(19) Europäisches Patentamt
European Patent Office
Office européen des brevets

(11) Publication number: 0 260 305 B1

# (12) EUROPEAN PATENT SPECIFICATION

(45) Date of publication of patent specification: 16.12.92

(51) Int. Cl.5: A61K 31/41, C07D 401/10

(21) Application number: 87902015.4

(22) Date of filing: 11.03.87

(86) International application number:
PCT/US87/00560

(87) International publication number:
WO 87/05510 (24.09.87 87/21)

## (54) OUINOLINYL ETHER TETRAZOLE AS AGENT FOR THE TREATMENT OF HYPERSENSITIVE AILMENTS.

(30) Priority: 13.03.86 US 839410
24.09.86 US 911028

(43) Date of publication of application:
23.03.88 Bulletin 88/12

(45) Publication of the grant of the patent:
16.12.92 Bulletin 92/51

(84) Designated Contracting States:
AT BE CH DE FR GB IT LI LU NL SE

(56) References cited:
EP-A- 0 200 101
US-A- 4 202 985
US-A- 4 474 788
US-A- 4 661 499

J.Med.Chem. 33, 2828-2841 (1990)

(73) Proprietor: RHONE-POULENC RORER INTERNATIONAL (HOLDINGS) INC.
Delaware Corporate Center 1, 1 Righter Parkway, Suite 114
Wilmington, Delaware(US)

(72) Inventor: YOUSSEFYEH, Raymond
435 Martling Avenue
Tarrytown, NY 10591(US)
Inventor: CHAKRABORTY, Utpal
12 Devonshire Court
Flemington, NJ 08822(US)
Inventor: MAGNIEN, Ernest
P.O. Box 1045
Norwich, VT 05055(US)
Inventor: DESAI, Rohit
1 Glenwood Road
Millwood, NY 10546(US)
Inventor: LEE, Thomas, D-Y
54 Vernon Drive
Scarsdale, NY 10583(US)

(74) Representative: Patentanwälte Grünecker, Kinkeldey, Stockmair & Partner
Maximilianstrasse 58
W-8000 München 22(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to 5-[3-methyl-4-(4-(2-quinolylmethyloxy)phenyl)butyl]tetrazole and its use as valuable pharmaceutical agent, particularly as lipoxygenase inhibitor and/or leukotriene antagonist possessing anti-inflammatory and anti-allergic properties.

EP-A-0200101 discloses aryl and heteroaryl ethers as agents for the treatment of hypersensitive aliments.

5-[3-methyl-4-(4-(2-quinolylmethyloxy(phenyl)butyl] tetrazole has the chemical formula

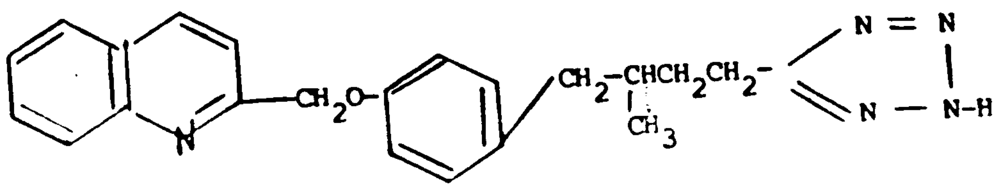

It can be prepared by art recognized procedures from known compounds or readily preparable intermediates, such as a method as described in the example.

Reaction temperatures are in the range of room temperature to reflux and reaction times vary from 2 to 48 h. The reaction is usually carried out in a solvent that dissolves both reactants and is inert to both as well.

Solvents include, for example diethyl ether, tetrahydrofuran, N,N-dimethyl formamide, dimethyl sulfoxide and dioxane.

The compound of the present invention may be prepared by Wittig reaction on the desired benzaldehyde. Thus for example the following schemes may be employed.

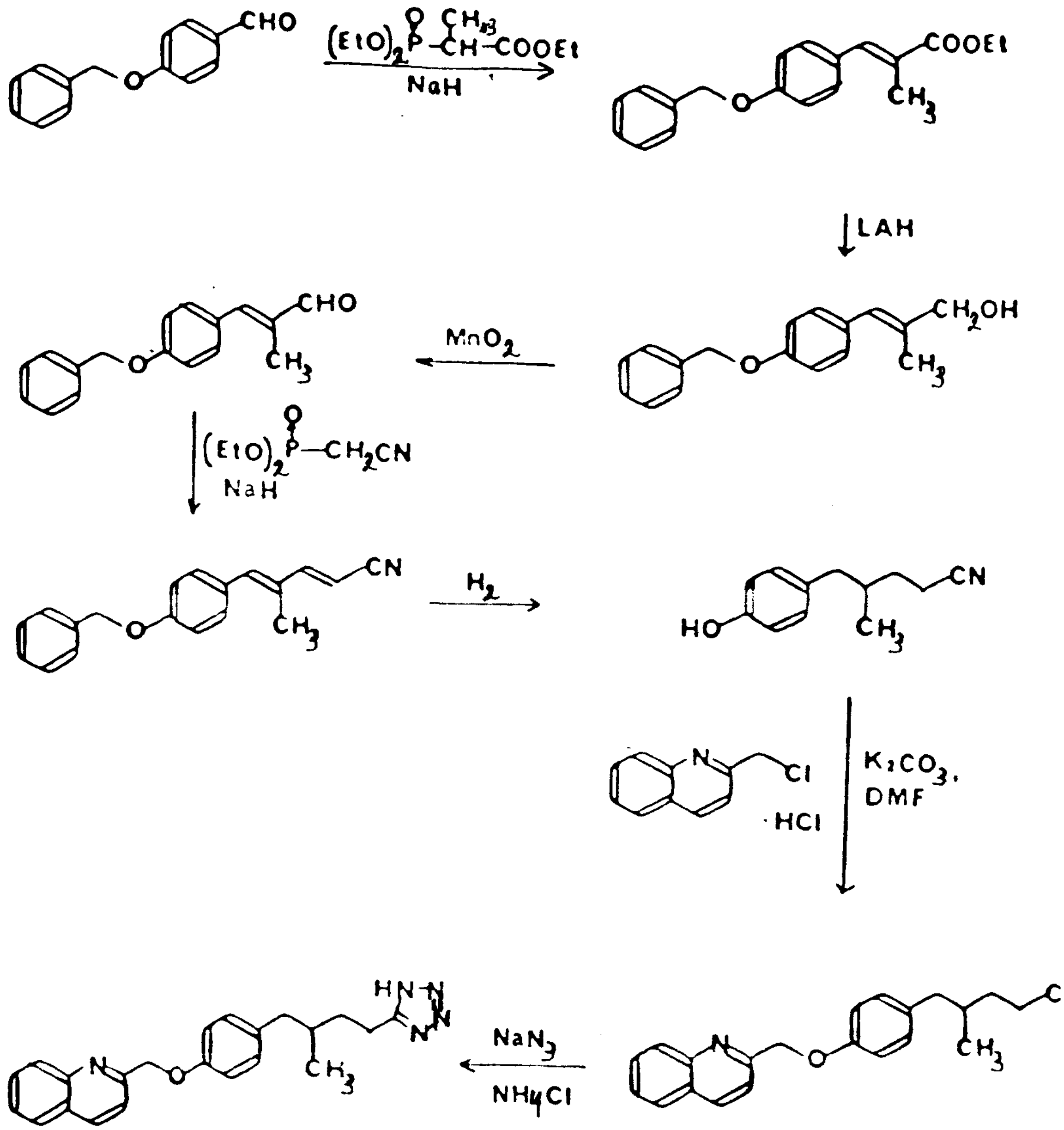


The tetrazole may be formed from the nitrile at various stages of the synthesis.

The compound of this invention may be obtained as racemic mixture of its dextro and levorotatory isomers since at least one asymmetric carbon atom is present. The product may exist as a mixture of two disastereomers based on syn and anti configurations. These diastereomers may be separated by fractional crystallization. Each diastereomer may then be resolved into dextro and levorotatory optical isomers by conventional methods.

Resolution may best be carried out in the intermediate stage where it is convenient to combine the racemic compound with an optically active compound by salt formation ester formation, or amide formation to form two disasteromeric products. If an acid is added to an optically active base, then two disastereomeric salts are produced which possess different properties and different solubilities and can be separated by fractional crystallization. When the salts have been completely separated by repeated crastallization, the base is split off by acid hydrolysis and the pure d and l acids are obtained.

The present compound forms salts with acids because a basic amino function is present. All such salts are useful in the isolation and/or purification of the compound of the present invention. Suitable acids include, for example, hydrochloric, sulfuric, nitric, benzenesulfonic, toluenesulfonic, acetic, malic and tartaric which are pharmaceutically acceptable.

The compound of the present invention has potent activity as leukotriene antagonists and as such

possesses therapeutic value in the treatment of inflammatory conditions and allergic responses such as anaphylaxis and asthma.

Protocol for SRS-A (slow reacting substance of anaphylaxis Antagonists

Leukotrienes, the products of the 5-lipoxygenase pathway of arachidonic acid metabolism, are potent contractile agents with a variety of smooth muscle preparations. Thus, it has been hypothesized that the leukotrienes contribute significantly to the pathophysiology of asthma. This protocol describes an *in vitro* assay used to test compounds which specifically antagonize the actions of leukotrienes.

Peripheral strips of guinea pig lungs are prepared and hung in tissue baths (Metro #ME-5505, 10 ml) according to the published procedure - (Proc. Nat'l. Acad. Sci., U.S.A. Volume 77, pp. 4354-4358, 1980). The strips are thoroughly rinsed in Assay Buffer and then connected with surgical silk thread support rods from the tissue baths. The rods are adjusted in the baths and the strips connected to the pressure transducers (Grass FT 103 or Gould US-3). The tissue baths are aerated with 95% oxygen - 5% carbon dioxide and maintained at 37°C. The assay buffer has been made as follows: for each l of buffer the following are added to approximately 800 ml of water distilled in glass-6.87 g NaCl, 0.4 g $MgSO_4.7H_2O$, and 2.0 g D-glucose. Then a solution of 0.368 g $CaCl_2.2H_2O$ in 100 ml glass-distilled water is slowly added to the buffer. Sufficient water is added to adjust the volume to 1 l, and the solution is aerated with 95% oxygen - 5% carbon dioxide. Usually 10 l of buffer are used for an experiment with 4 tissues. After the tissues have been repeatedly washed and allowed to equilibrate in the tissue bath, they are challenged with 1 M histamine. After maximum contractions have been obtained, the tissues are washed, and allowed to relax back to baseline tension. This histamine challenge procedure is repeated at least 1 to 2 more times to obtain a repeatable control response. The average response to 1 µM histamine for each tissue is used to normalize all other challenges.

Responses of each tissue to a predetermined concentration of leukotriene are then obtained. Usually test compounds are examined initially at 30 µM on resting tension of the tissues without any added agaonist or antagonist to determine if the compound has any possible intrinsic activity. The tissues are washed and the test compound is added again. Leukotriene is added after the desired preincubation time. The intrinsic activity of the compounds, and their effect on leikotriene-induced contractions are then recorded.

The results of this test for the compound of the this invention indicates that this compound is considered to be useful leukotriene antagonists.

Inhibitions of ($^3$H)-$LTD_4$ Binding Membranes from Guinea Pig Lung.

A. Preparation of the Crude Receptor Fraction:

This procedure was adapted from Mong *et al.* 1984). Male guinea pigs are sacrificed by decapitation and their lungs are quickly removed and placed in a beaker containing ice-cold homgenization buffer. The lungs are separated from connective tissue, minced with scissors, blotted dry and weighed. The tissue is then homogenized in 40 volumes (w/v) of homogenization buffer with a Polytron at a setting of 6 for 30 s. The homogenate is centrifuged at 1000 x g for 10 min (e.g. 3500 RPM, SS-34 Rotor). The supernate is filtered through two layers of cheese cloth and centrifuged at 30,000 x g for 30 min (e.g. 18,500 RPM SS-34 Rotor), after which the resulting pellet is resuspended in 20 volumes of assay buffer by hand homoginization using a Dounce homogenizer. The final pellet is resuspended in 10 volumes of assay buffer and kept at 4°Cuntil use.

B. Binding Assay:

Each assay tube (16 x 100 mm) contains the following:

490 l Assay Buffer

10 l Test compound or solvent

100 l $^3$H-$LTD_4$ (*ca.* 17,500 DMP)

400 l Protein preparation

Incubations are done at 25°C for 20 min in a shaking water bath. Reactions are started by the addition of the protein preparation. At the end of the incubation time, 4.0 ml of cold wash buffer is added to the tube. After being vortexed, the contents of the tube are immediately poured over a Whatman GF/C Filter (25 mm diameter) which is sitting in a vacuum manifold (e.g., Millipore Model No. 3025 manifold) to which a partial

vacuum is applied. The filters are immediately washed with an additional 15 ml of cold buffer. The filters are transferred to 7 ml plastic scintillation vials to which 6.0 ml of appropriate scintillation fluid (e.g., Scintiverse) is added. After being allowed to quilibrate for 4-6 h, the readioactivity is counted with a liquid scintillation counter appropriately set for tritium.

The required control assay tubes include the following:

(a) Total Binding: No test compound is added; buffer is substituted.

(b) Non-Specific Binding: Non-labeled ligand is added at a concentration of 1 μM.

(c) Solvent Controls: If test compound is dissolved in a solvent, controls for both Total Binding and Non-Specific Binding containing solvent but no compounds are required.

The results of this test indicate that the compound of this invention exhibits valuable properties which are useful in the treatment of inflammatory conditions and allergic responses.

The compound of the present invention can be administered to a mammalian host in a variety of forms adapted to the chosen route of administration, i.e., orally, or parenterally. Parenteral administration in this respect includes administration by the following routes: intravenous, intramuscular, subcutaneous, intraocular, intrasynovial, transepthelially including transdermal, opthalmic, sublingual and buccal; topically includes opthalmic, dermal, ocular, rectal and nasal inhalation via insufflation and aerosol and rectal systemic.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of, for example, ingestible tablets, buccal tablets, troches, capsules, elixirs, suspensions, syrups and wafers. Such compositions and preparations should contain at least 0.1% of the active compound. The percentage of the compositions and preparations may, of course, be varied and may conveniently be between about 2 to about 6% of the weight of the unit. The amount of active compound in such therapeutically useful compositions is such that a suitable dosage will be obtained. Preferred compositions or preparations according to the present invention are prepared so that an oral dosage unit form contains between about 50 and 300 mg of active compound.

The tablets, troches, pills or capsules may also contain the following: A binder such as gum tragacanth, acacia, corn starch or gelatin; excipients such as dicalcium phosphate; a disintegrating agent such as corn starch, potato starch or alginic acid; a lubricant such as magnesium stearate; and a sweetening agent such as sucrose, lactose or saccharin may be added, or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens a preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersion can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It may be stable under the conditions of manufacture and storage and must be preserved against the contaminating action of microorganisms such as bacteria and fungi. The carrier can be a solvent or dispersion medium containing, for example, wate, ethanol, polyol (for example, glycerol, propylene glycol, and liquid polyethylene glycol), suitable mixtures thereof, and vegetable oils. The proper fluidity can be maintained , for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimersal. In many cases, it will be preferable to include isotonic agents, for example, sugars or Sodium chloride. Prolonged absorption of the injectable compositions can be brought about by the use in the compositions of agents delaying absorption, for example, aluminum monostearate and

gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those anumerated above. In the case of sterile powders for the preparation of sterile injectable solutions, the preferred methods of preparation are vacuum drying and the freeze drying technique which yield a powder of the active ingredient plus any additional desired ingredient from previously sterile-filtered solution thereof.

The therapeutic compound of this invention may be administered to a mammal alone or in combination with pharmaceutically acceptable carriers, as noted above, the proportion of which is determined by the solubility and chemical nature of the compound, chosen route of administration and standard pharmaceutical practice.

The physician will determine the dosage of the present therapeutic agent which will be most suitable for prophylaxis or treatment and it will vary with the form of administration and the particular compound chosen, and also, it will vary with the particular patient under treatment. He will generally wish to initiate treatment with small dosages by small increments until the optimum effect under the circumstances is reached. The therapeutic dosage will generally be from 0.1 to 100 µM/day or from about 0.1 mg to about 50 mg/kg of body weight per day and higher although it may be administered in several different dosage units. Higher dosages are required for oral administration.

The compound of the present invention may be prepared by the following example.

EXAMPLE

5-[3-METHYL-4-(4-(2-QUINOLYLMETHYLOXY)PHENYL)BUTYL]TETRAZOLE

A. 4-benzyloxy-α-methyl-cinnamic acid ethyl ester. To a solution of sodium hydride (60% oil dispersion, 3.1 g) and triethyl 2-phosphonopropionate (15.5 g) in tetrahydrofuran (50 ml) is added dropwise a tetrahydrofuran solution of 4-benzyloxybenzaldehyde (10.6 g). After stirring at room temperature for 2 h, the reaction mixture is poured into ice water. The insoluble yellowish solid is collected, purified and used directly in the next step.

B. 4-benzyloxy-α-methyl-cinnamic alcohol. Under argon and with stirring, a tetrahydrofuran solution of 4-benzyloxy-α-methyl-cinnamic acid ethyl ester (11.9 g) is added dropwise to a cooled tetrahydrofuran solution of lithium aluminum hydride (2.5 g). The reaction mixture is allowed to stir for 18 h, and afterwards, the excess reagent is destroyed in a conventional manner. The residue which results from the evaporation of the solvent is partitioned in a water/ethyl acetate mixture and from the organic layer, there is obtained 7.8 g of a white solid of the desired product. This is used directly in the next step.

C. 4-benzyloxy-α-methyl-cinnamyl aldehyde. Manganese dioxide (15 g total) is added portionwise to a dichloromethane solution (100 ml) of 4-benzyloxy-αmethyl-cinnamic alcohol with stirring over a period of one week. After two filtrations, the filtrate is evaporated to yield a gum. Upon treatment with cold hexane, 4.8 g of product is obtained as white granules and used directly in the next step.

D. 5-(p-benzyloxyphenyl)-4-methyl-2,4-penta-dienenitrile. To a solution of sodium hydride (60% oil dispersion, 1.5 g) and diethyl cyanomethylphosphonate (5.4 g) in tetrahydrofuran (50 ml) is added dropwise a tetrahydrofuran solution of 4-benzyloxy-α-methyl-cinnamyl aldehyde (4.8 g). After stirring at room temperature for 2 h, the reaction mixture is poured into ice water. The insoluble material is collected to obtain 4.6 g of off-white solid, after purification this is used directly in the next step.

E. 5-p-hydroxyphenyl-4-methylvaleronitrile. 5-(p-Benzyloxyphenyl)-4-methyl-2,4-pentadienenitrile (4.3 g) (4.3 g) dissolved in ethanol is hydrogenated (0.8 g of 5% palladium over charcoal as catalyst) around 207 k pa 30 psi overnight. After filtering off the catalyst, the solvent is evaporated to give an oil (2.9 g). This is used directly in the next step.

F. 4-methyl-5-[4-(2-quinolylmethyloxy)phenyl]valeronitrile. A reaction mixture of 5-p-hydroxyphenyl-4-methyl-valeronitrile (2.9 g, 2-chloromethylquinoline hydrochloride (4.2 g) and anhydrous potassium carbonate (30 g) in dimethylformamide (60 ml) is stirred and heated (110°C) for 5 h. Afterward, the solvent is removed under vaccuum and the residue is partitioned in a mixture of chloroform/water. The organic layer is evaporated and the resultant oil is purified on a silica gel dry column (chloroform as eluant) to give 2.3 g of an off-white solid. This is used directly in the next step.

G. 5-[3-methyl-4-(4-(2-quinolylmethyloxy)phenyl)-butyl]tetrazole. A mixture of 4-methyl-5-[4-(2-quinoly]-methyloxy) phenyl]valeronitrile (1.5 g), sodium azide (3 g), ammonium chloride (2.8 g) in dimethylformamide (20 ml) is stirred and heated at 135°C for 18 h. After cooling, the reaction mixture is poured into ice water and the insoluble material is taken up by chloroform. The residue from the evaporation of chloroform is purified by silica gel dry column (5% methanol in chloroform as eluant) twice followed by trituration with ether/hexane to yield 5-[3-methyl-4-(4-(2-quinolylmethyloxy)phenyl)butyl]-tetrazole. (0.7 g m.p. 75-77°C).

## Claims

### Claims for the following Contracting States : BE, CH, DE, FR, GB, IT, LI, LU, NL, SE

**1.** A compound of the formula

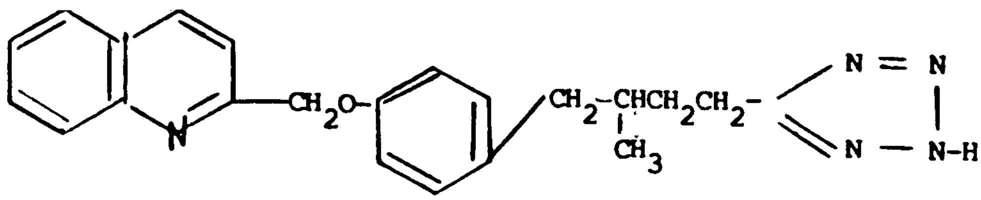

and pharmaceutically acceptable salts thereof.

**2.** A process for preparing the compound of claim 1 comprising

a) reacting 4-benzyloxybenzaldehyde with sodium hydride and triethyl-2-phosphonopropionate in a solvent to obtain 4-benzyloxy-α-methyl-cinnamic acid ethyl ester,

b) reacting 4-benzyloxy-α-methyl-cinnamic acid ethyl ester with lithium aluminum hydride in a solvent to obtain 4-benzyloxy-α-methyl-cinnamic alcohol,

c) reacting 4-benzyloxy-α-methyl-cinnamic alcohol with manganese dioxide in a solvent to obtain 4-benzyloxy-α-methyl-cinnamyl aldehyde,

d) reacting 4-benzyloxy-α-methyl-cinnamyl aldehyde with sodium hydride and diethyl cyanomethylphosphonate to obtain 5-(p-benzyloxyphenyl)-4-methyl-2,4-pentadienenitrile,

e) hydrogenating 5-(p-benzyloxyphenyl)-4-methyl-2,4-pentadienenitrile in a solvent to obtain 5-p-hydroxyphenyl-4-methyl-valeronitrile,

f) reacting 5-p-hydroxyphenyl-4-methyl-valeronitrile with potassium carbonate in a solvent to obtain 4-methyl-5-[4-(2-quinolylmethyloxy)phenyl]-valeronitrile,

g) reacting 4-methyl-5-[4-(2-quinolylmethyloxy)phenyl] valeronitrile with sodium azide and ammonium chloride in a solvent to obtain 5-[3-methyl-4-(4-(2-quinolylmethyloxy)phenyl)butyl]tetrazole.

**3.** A pharmaceutical composition comprising the compound of claim 1 as active ingredient, optionally in admixture with a pharmaceutical carrier and/or diluent.

### Claims for the following Contracting State : AT

**1.** A process for preparing a compound of the formula

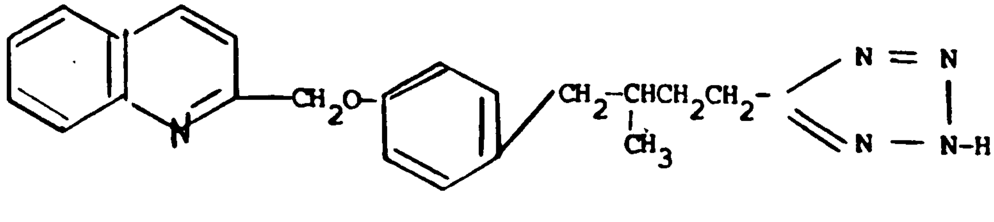

comprising

a) reacting 4-benzyloxybenzaldehyde with sodium hydride and triethyl-2-phosphonopropionate in a solvent to obtain 4-benzyloxy-α-methyl-cinnamic acid ethyl ester,

b) reacting 4-benzyloxy-α-methyl-cinnamic acid ethyl ester with lithium aluminum hydride in a solvent to obtain 4-benzyloxy-α-methyl-cinnamic alcohol,

c) reacting 4-benzyloxy-α-methyl-cinnamic alcohol with manganese dioxide in a solvent to obtain 4-benzyloxy-α-methyl-cinnamyl aldehyde,
d) reacting 4-benzyloxy-α-methyl-cinnamyl aldehyde with sodium hydride and diethyl cyanomethylphosphonate to obtain 5-(p-benzyloxyphenyl)-4-methyl-2,4-pentadienenitrile,
e) hydrogenating 5-(p-benzyloxyphenyl)-4-methyl-2,4-pentadienenitrile in a solvent to obtain 5-p-hydroxyphenyl-4-methyl-valeronitrile,
f) reacting 5-p-hydroxyphenyl-4-methyl-valeronitrile with potassium carbonate in a solvent to obtain 4-methyl-5-[4-(2-quinolylmethyloxy)phenyl]-valeronitrile,
g) reacting 4-methyl-5-[4-(2-quinolylmethyloxy)phenyl] valeronitrile with sodium azide and ammonium chloride in a solvent to obtain 5-[3-methyl-4-(4-(2-quinolylmethyloxy)phenyl)butyl]tetrazole.

**2.** A process for preparing a pharmaceutical composition comprising formulating the compound of claim 1 as active ingredient, optionally by mixing with a pharmaceutical carrier and/or diluent.

**Patentansprüche**

**Patentansprüche für folgende Vertragsstaaten : BE, CH, DE, FR, GB, IT, LI, LU, NL, SE**

**1.** Verbindung der Formel

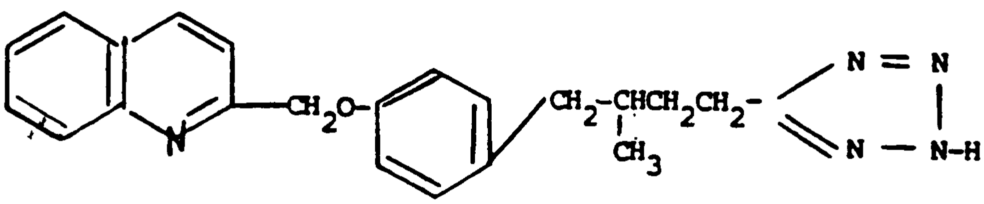

und pharmazeutisch annehmbare Salze davon.

**2.** Verfahren zur Herstellung der Verbindung nach Anspruch 1, umfassend
a) die Umsetzung von 4-Benzyloxybenzaldehyd mit Natriumhydrid und Triethyl-2-phosphonoproprionat in einem Lösungsmittel, um 4-Benzoyloxy-α-methyl-zimtsäureethylester zu erhalten,
b) die Umsetzung von 4-Benzyloxy-α-methyl-zimtsäureethylester mit Lithiumaluminiumhydrid in einem Lösungsmittel, um 4-Benzyloxy-α-methyl-zimtalkohol zu erhalten,
c) die Umsetzung von 4-Benzyloxy-α-methyl-zimtalkohol mit Mangandioxid in einem Lösungsmittel, um 4-Benzyloxy-α-methyl-zimtaldehyd zu erhalten,
d) die Umsetzung von 5-Benzyloxy-α-methyl-zimtaldehyd mit Natriumhydrid und Diethylcyanomethylphosphonat, um 5-(p-Benzyloxyphenyl)-4-methyl-2,4-pentadiennitril zu erhalten,
e) die Hydrierung von 5-(p-Benzyloxyphenyl)-4-methyl-2,4-pentadiennitril in einem Lösungsmittel, um 5-p-Hydroxyphenyl-4-methylvaleronitril zu erhalten,
f) die Umsetzung von 5-p-Hydroxyphenyl-4-methylvaleronitril mit Kaliumcarbonat in einem Lösungsmittel, um 4-Methyl-5-[4-(2-chinolylmethyloxy)phenyl]valeronitril zu erhalten,
g) die Umsetzung von 4-Methyl-5-[4-(2-chinolylmethyloxy)phenyl]-valeronitril mit Natriumazid und Ammoniumchlorid in einem Lösungsmittel, um 5-[3-Methyl-4-(4-(2-chinolylmethyloxy)phenyl)butyl]-tetrazol zu erhalten.

**3.** Pharmazeutische Zusammensetzung, umfassend die Verbindung nach Anspruch 1 als aktiven Bestandteil, gegebenenfalls in Mischung mit einem pharmazeutischen Träger und/oder Verdünnungsmittel.

**Patentansprüche für folgenden Vertragsstaat : AT**

**1.** Verfahren zur Herstellung einer Verbindung der Formel

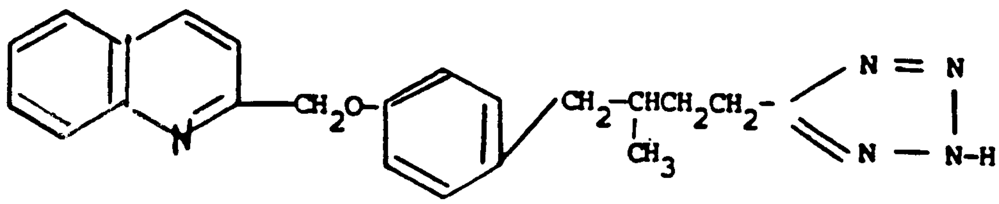

umfassend

a) die Umsetzung von 4-Benzyloxybenzaldehyd mit Natriumhydrid und Triethyl-2-phosphonoproprionat in einem Lösungsmittel, um 4-Benzoyloxy-α-methyl-zimtsäureethylester zu erhalten,
b) die Umsetzung von 4-Benzyloxy-α-methyl-zimtsäureethylester mit Lithiumaluminiumhydrid in einem Lösungsmittel, um 4-Benzyloxy-α-methyl-zimtalkohol zu erhalten,
c) die Umsetzung von 4-Benzyloxy-α-methyl-zimtalkohol mit Mangandioxid in einem Lösungsmittel, um 4-Benzyloxy-α-methyl-zimtaldehyd zu erhalten,
d) die Umsetzung von 5-Benzyloxy-α-methyl-zimtaldehyd mit Natriumhydrid und Diethylcyanomethylphosphonat, um 5-(p-Benzyloxyphenyl)-4-methyl-2,4-pentadiennitril zu erhalten,
e) die Hydrierung von 5-(p-Benzyloxyphenyl)-4-methyl-2,4-pentadiennitril in einem Lösungsmittel, um 5-p-Hydroxyphenyl-4-methylvaleronitril zu erhalten,
f) die Umsetzung von 5-p-Hydroxyphenyl-4-methylvaleronitril mit Kaliumcarbonat in einem Lösungsmittel, um 4-Methyl-5-[4-(2-chinolylmethyloxy)phenyl]valeronitril zu erhalten,
g) die Umsetzung von 4-Methyl-5-[4-(2-chinolylmethyloxy)phenyl]-valeronitril mit Natriumazid und Ammoniumchlorid in einem Lösungsmittel, um 5-[3-Methyl-4-(4-(2-chinolylmethyloxy)phenyl)butyl]-tetrazol zu erhalten.

**2.** Verfahren zur Herstellung einer pharmazeutischen Zusammensetzung, umfassend die Formulierung der Verbindung nach Anspruch 1 als aktiven Bestandteil, gegebenenfalls durch Mischen mit einem pharmazeutischen Träger und/oder Verdünnungsmittel.

## Revendications
**Revendications pour les Etats contractants suivants : BE, CH, DE, FR, GB, IT, LI, LU, NL, SE**

**1.** Composé représenté par la formule :

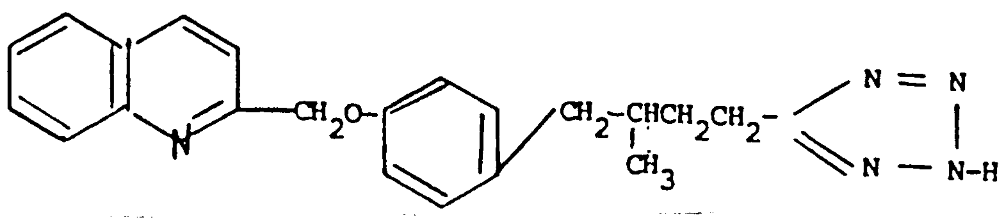

et sels pharmaceutiquement acceptables de ce composé.

**2.** Procédé de préparation du composé tel que défini à la revendication 1, comprenant :

(a) la réaction du benzyloxy-4 benzaldéhyde avec de l'hydrure de sodium et du phosphono-2 propionate de triéthyle, dans un solvant, pour obtenir l'ester éthylique de l'acide benzyloxy-4 α-méthyl-cinnamique ;
(b) la réaction de l'ester éthylique de l'acide benzyloxy-4 α-méthyl-cinnamique avec de l'hydrure de lithium et d'aluminium, dans un solvant, pour obtenir de l'alcool benzyloxy-4 α-méthyl-cinnamique ;
(c) la réaction de l'alcool benzyloxy-4 α-méthyl-cinnamique avec du dioxyde de manganèse, dans un solvant, pour obtenir de l'aldéhyde benzyloxy-4 α-méthylcinnamylique ;
(d) la réaction de l'aldéhyde benzyloxy-4 α-méthylcinnamylique avec de l'hydrure de sodium et du cyanométhylphosphonate de diéthyle, pour obtenir du (p-benzyloxyphényl)-5 méthyl-4 pentadiène-2,4 nitrile ;
(e) l'hydrogénation du (p-benzyloxyphényl)-5 méthyl-4 pentadiène-2,4 nitrile, dans un solvant, pour obtenir du p-hydroxyphényl-5 méthyl-4 valéronitrile ;
(f) la réaction du p-hydroxyphényl-5 méthyl-4 valéronitrile avec du carbonate de potassium, dans un

solvant, pour obtenir du méthyl-4 [(quinolyl-2 méthyloxy)-4 phényl]-5 valéronitrile ;
(g) la réaction du méthyl-4 [(quinolyl-2 méthyloxy)-4 phényl]-5 valéronitrile avec de l'azide de sodium et du chlorure d'ammonium, dans un solvant, pour obtenir du [méthyl-3 (quinolyl-2 méthyloxy)-4 phényl)-4 butyl]-5 tétrazole.

**3.** Composition pharmaceutique comprenant le composé tel que défini à la revendication 1 en tant qu'ingrédient actif, facultativement en mélange avec un support et/ou diluant pharmaceutique.

**Revendications pour l'Etat contractant suivant : AT**

**1.** Procédé de préparation d'un composé représenté par la formule :

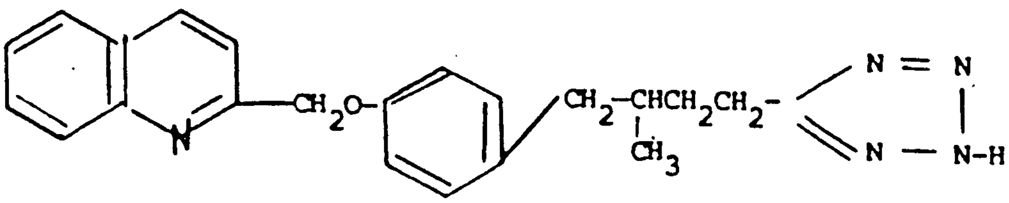

comprenant :
(a) la réaction du benzyloxy-4 benzaldéhyde avec de l'hydrure de sodium et du phosphono-2 propionate de triéthyle, dans un solvant, pour obtenir l'ester éthylique de l'acide benzyloxy-4 α-méthyl-cinnamique ;
(b) la réaction de l'ester éthylique de l'acide benzyloxy-4 α-méthyl-cinnamique avec de l'hydrure de lithium et d'aluminium, dans un solvant, pour obtenir de l'alcool benzyloxy-4 α-améthyl-cinnamique ;
(c) la réaction de l'alcool benzyloxy-4 α-méthyl-cinnamique avec du dioxyde de manganèse, dans un solvant, pour obtenir de l'aldéhyde benzyloxy-4 α-méthyl-cinnamylique ;
(d) la réaction de l'aldéhyde benzyloxy-4 α-méthyl-cinnamylique avec de l'hydrure de sodium et du cyanométhylphosphonate de diéthyle, pour obtenir du (p-benzyloxyphényl)-5 méthyl-4 pentadiène-2,4 nitrile ;
(e) l'hydrogénation du (p-benzyloxyphényl)-5 méthyl-4 pentadiène-2,4 nitrile, dans un solvant, pour obtenir du p-hydroxyphényl-5 méthyl-4 valéronitrile ;
(f) la réaction du p-hydroxyphényl-5 méthyl-4 valéronitrile avec du carbonate de potassium, dans un solvant, pour obtenir du méthyl-4 [(quinolyl-2 méthyloxy)-4 phényl]-5 valéronitrile ;
(g) la réaction du méthyl-4 [(quinolyl-2 méthyloxy)-4 phényl]-5 valéronitrile avec de l'azide de sodium et du chlorure d'ammonium, dans un solvant, pour obtenir du [méthyl-3((quinolyl-2 méthyloxy)-4 phényl)-4 butyl]-5 tétrazole.

**2.** Procédé de préparation d'une composition pharmaceutique comprenant la formulation du composé tel que défini à la revendication 1 en tant qu'ingrédient actif, facultativement par mélange avec un support et/ou diluant pharmaceutique.